# EUROPEAN PATENT SPECIFICATION

(11) **EP 0 357 127 B1**
(45) Date of publication and mention of the grant of the patent: **09.06.1999**
(21) Application number: 89202106.4
(22) Date of filing: 16.08.1989
(51) Int. Cl.: C12N 15/80, C12N 15/90, C12P 21/02, C12N 1/15

(54) **Gene replacement as a tool for the construction of aspergillus strains**
Genaustausch als Mittel zur Konstruktion von Aspergillus-Stämmen
Remplacement de gènes comme règle de construction de souches d'aspergillus

(30) Priority: 16.08.1988 EP 88201743
(43) Date of publication of application: 07.03.1990
(73) Proprietor: DSM N.V., 6411 TE Heerlen (NL)
(72) Inventor: Van Hartingsveldt, Wim, NL-2613 DL Delft (NL); Van den Hondel, Cees A.M.J.J., NL-2804 PZ Gouda (NL); Veenstra, Annemarie Eveline, NL-2151 XH Nieuw Vennep (NL); Van den Berg, Johannes A., NL-2811 AD Reeuwijk (NL)
(74) Representative: Swinkels, Bart Willem

(56) References cited:
- EP-A- 0 244 234
- EP-A- 0 249 350
- BIOTECHNOLOGY, vol. 5, no. 12, December 1987, pages 1301-1304, New York, NY, US; A. UPSHALL et al.: "Secretion of active human tissue plasminogen activator from the filamentous fungus Aspergillus nidulans"
- GENE, vol. 63, no. 2, 1988, pages 199-212, Elsevier Science Publishers B.V., Amsterdam, NL; M.A. DAVIS et al.: "An amdS-lacZ fusion for studying gene regulation in Aspergillus"
- BIOTECHNOLOGY, vol. 5, no. 4, April 1987, pages 369-376, New York, NY, US; D. CULLEN et al.: "Controlled expression and secretion of bovine chymosin in Aspergillus nidulans"
- BIOTECHNOLOGY, vol. 6, no. 4, April 1988, pages 417-421, New York, NY, US; G.E. COLE et al.: "Stable expression of Aspergillus awamori glucoamylase in distiller's yeast"

## Description

### INTRODUCTION

### Technical Field

This invention relates to transformation and expression employing filamentous fungal transformants.

### Background

The ability to transform viable cells with DNA capable of expression has opened numerous avenues to new and improved products. This capability has allowed the production of a large number of mammalian proteins, which were otherwise not readily accessible. In other instances, particularly blood proteins, the need to use natural sources for the production of such proteins became increasingly unacceptable with the widespread problem of hepatitis and AIDS. Besides mammalian proteins for use as drugs, there are a number of other proteins, particularly enzymes, which find application in a number of commercial processes. Chymosin may be used in the production of cheese, lipases find employment inter alia in transesterification reactions, proteases find employment inter alia in food and medical applications, and the like.

In producing these various products, there is a strong interest in the economics of the production of the product. The first aspect of concern is the level of expression of the desired product. A second concern is the stability of the product in the particular host. A third concern is the isolation and purification of the product, particularly where the product may be a drug or food additive.

In producing the protein product, depending upon a functional signal sequence, the product may be retained in the cytoplasm or be secreted into the nutrient medium. Different hosts have different secretion products, as well as varying levels of secretion, where the secretion may be inducible or non-inducible. In most situations, secretion offers many advantages, since the product may be isolated from the nutrient medium substantially free of intracellular proteins and cellular debris. Thus, the separation would only have to be subject to the other proteins secreted by the host and the small amounts of protein which may result from cell lysis or degradation. It is therefore of substantial interest to provide systems which result in efficient expression and secretion of products of interest under conditions for easy isolation and purification.

### Relevant Literature

Transformation of filamentous fungi has been described by several authors (Tilburn et al., Gene 26 (1983) 205-221; John and Peberdy, Enzyme Microb. Technol. 6 (1984) 386-389; Ballance and Turner, Gene 36 (1985) 321-331; Van Hartingsveldt et al., Mol. Gen. Genet. 206 (1987) 71-75; Goosen et al., Curr. Genet. 11 (1987) 499-503); in EP-A-0134438 and 0238023 and International Patent Application (PCT) WO 86/06097.

Fungal species such as Aspergillus niger, Aspergillus oryzae, Mucor miehei, and Trichoderma reesei are used widely in the industrial production of enzymes, e.g. for use in the food industry (See for example, Strijkert, Antonie van Leeuwenhoek 53 (1987) 357-362). Their use is based on the secretory capacity of these microorganisms. A. niger and A. oryzae are used in large quantities and on a commercial scale for this industrial production and are consequently well characterized microorganisms with respect to their fermentation behaviour. Genetic engineering techniques have been applied to Aspergilli and transformants have been obtained that synthesize additional useful products (Cullen et al., Bio/technology 5 (1987) 369-376; Gwynne et al., Bio/technology 5 (1987) 713-719; Upshall et al., Bio/technology 5 (1987) 1301-1304). In these cases the selected protein has been expressed in addition to the proteins normally secreted by the host and hence the selected protein needs to be separated from those.

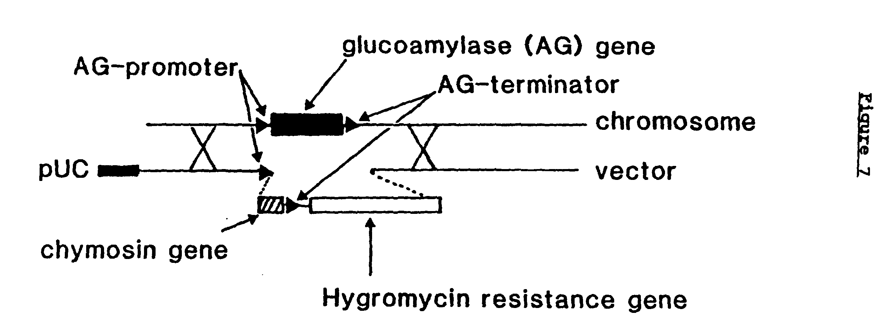

Gene replacement has been documented for Aspergilli (e.g., Miller et al., Mol. Cell. Biol. 5 (1985) 1721; Van Hartingsveldt et al., vide supra; Goosen et al., Curr. Genet. 11 (1987) 499-503; Wernars et al., Mol. Gen. Genet. 209 (1987) 71-77). For A. nidulans, for genes encoding enzymes that form part of biosynthetic pathways, e.g. for amino acids and nucleotides, various methods have been employed. Miller et al. (vide supra) use either a one step gene replacement of the TrpC gene with a restriction fragment containing only a modified, homologous gene, or a two step process using a circular plasmid. "One step" gene replacement requires the occurrence of two crossing over events in one single gene replacement (cf. Figure 7); in a two step process the first step is a single integration event, followed by a recombination that leaves the integrated gene copy and removes the native one (second step). The occurrence of the second step has been selected by screening for the phenotype of the integrated gene copy.

It is known that the natural expression level of many genes in the amino acid metabolic pathway, e.g., TrpC and ArgB, is at only a moderate level. The enzymes encoded by these genes form only a very small fraction of the total cell protein. However, these genes are essential, since their loss results in auxotrophy and the necessity to supply the metabolite or amino acid to the growth medium. Therefore, both of these gene replacements and gene replacement of analogous genes are easily achieved and transformants can be easily isolated (Esser and Mohr, Process Biochemistry (1986) 153-159).

For A. niger, similar results have been reported by Van Hartingsveldt et al., vide supra and Goosen et al., vide supra for the pyrG locus. The pyrG gene encodes an enzyme from the biosynthetic pathway leading to uridine. In these examples, gene replacement was again in an easily selectable gene, i.e., the gene that was used as a selection marker in the transformation.

It is to be noted that none of the obtained proteins described in the above references was secreted, either by A. nidulans or by A. niger.

EP-A-24434 describes the inactivation of the T. reesei cbh1 gene, which encodes an activity secreted enzyme. The inactivation is achieved by insertion of a plasmid containing a truncated version of the cbh1 cDNA into the cbh1 gene, but without the concomitant expression of a secretory gene in the inactivated cbh1 locus.

There is, therefore, a need to provide gene replacement systems for genetic loci which are actively expressed and encode proteins forming a major part of the total cell protein, and for genetic loci which encode proteins which are actively secreted by the cell.

### SUMMARY OF THE INVENTION

Expression systems for filamentous fungi are provided employing secretory-efficient hosts with cassettes devised for homologous recombination at a locus of a sequence encoding a highly expressed and secreted protein.

Conveniently, integration may include a marker protein, where the expression cassette is bordered by the 5' and 3' non-coding regions of the target gene. The transformant provides for high-levels of expression and efficient secretion. Optionally, a protease inhibitor is maintained in the nutrient medium to prevent proteolysis of the desired product.

### BRIEF DESCRIPTION OF THE DRAWINGS

Figure 1 depicts the oligonucleotides used to isolate the A. niger glucoamylase (AG) gene;
Figure 2 is a map of the A. niger glucoamylase (AG) gene, of subclones that have been constructed and of a hybridization probe used in the experiments;
Figure 3 depicts the construction of pAB64-40;
Figure 4 is a schematic representation of pAN76-1;
Figure 5 is a schematic representation of mAB64-0;
Figure 6 depicts schematic representations of pAB64-72, pAB64-73 and pAB64-75; and
Figure 7 is a schematic representation of gene
   EP-A-24434 describes the inactivation of the T. reesei cbh 1 gene, which encodes an activity secreted enzyme. The inactivation is achieved by insertion of a plasmid containing a truncated version of the cbh 1 cDNA into the cbh 1 gene, but without the concomitant expression of a secretory gene in the inactivated cbh 1 locus. replacement at the glucoamylase locus.

### DESCRIPTION OF THE SPECIFIC EMBODIMENTS

Efficient expression systems are provided employing filamentous fungi. The systems employ secretory hosts for integration of expression constructs. The expression constructs comprise the gene of interest, which for homologous recombination are bordered by 5' and 3' non-coding regions of the target locus, for integration. The target locus will comprise a gene encoding a secretory protein. Growth and induction of the transformant host results in efficient expression and secretion of the desired product, as well as ease of isolation, in view of the absence of the expression product of the target locus in the nutrient medium.

The hosts of interest are those filamentous fungi which have efficient levels of secretion of their endogenous proteins. Industrial production strains of Aspergillus, particularly niger, awamori or oryzae, are of particular interest. Alternatively, Trichoderma reesei, M. miehei and aspergilli, such as A. ficuum may be employed.

The expression construct will comprise the gene of interest, usually having a signal sequence which is functional in the host and provides for secretion of the expression product. For the most part for homologous recombination, the signal sequence will be homologous or substantially homologous with the signal sequence of the gene at the target locus. However, signal sequences can be designed to provide for secretion. For example, see Von Heijne, Eur. J. Biochem. 133 (1983) 17-21; and Perlman and Halvorson, J. Mol. Biol. 167 (1983) 391-409. The expression product or gene of interest may be homologous or heterologous to the host. By "homologous" is intended a protein native to the wild type host, while "heterologous" is intended a protein which is foreign to the host and includes mutants which are not encountered in nature.

The gene of interest may have its own signal sequence or be joined to a signal sequence of the host or a synthetic signal sequence, as appropriate. The particular signal sequence which is chosen may vary, depending upon the gene of interest and the target locus. Of particular interest is to employ the signal sequence of the target locus which provides for an additional region of homology for integration at that site and which is known to allow for efficient secretion. The DNA sequence encoding the signal sequence may be joined directly through the sequence encoding the processing signal (cleavage recognition site) to the sequence encoding the mature protein or through a short bridge, usually fewer than ten codons.

The region 5' to the open reading frame for the gene of interest will comprise the transcriptional initiation regulatory region. Any region functional in the host may be employed. However, for the most part, the region which is employed will be homologous with the region of the target locus. This has effect of substituting the expression product of the target locus with the expression product of interest. To the extent that the level of expression and secretion of the endogenous protein provides for efficient production, this transcription initiation regulatory region will normally be found to be satisfactory. However, in some instances, one may wish a higher level of transcription than the wild type gene or one may wish to have inducible expression employing a particular inducing agent. In those instances, a transcriptional initiation regulatory region will be employed which is different from the region at the target locus. A large number of transcriptional initiation regulatory regions are known which are functional in filamentous fungi. These regions include the genes encoding glucoamylase, fungal amylase, acid phosphatase, GAPDH, TrpC, AmdS, AlcA, AldA, histone H2A, Pyr4, PyrG, isopenicillin N synthetase, PGK, acid protease, acyl transferase, and the like.

The target locus will preferably encode a highly expressed protein gene, i.e., a gene whose expression product is secreted to a concentration of at least about 0.1 g/l at the end of the fermentation process. The duration of this process may vary depending inter alia on the protein product desired. As an example of such a gene, the gene encoding glucoamylase (AG) is illustrative. Other genes of interest include fungal α-amylase, acid phosphatase, protease, acid protease, lipase, phytase and cellobiohydrolase.

The gene of interest may be any gene which may have some intended application. Selected proteins, heterologous to Aspergillus, are e.g., chymosin, including its precursor forms (such as prochymosin, preprochymosin and pseudochymosin), interleukins, blood clotting factors, such as factor VIII or IX, phospholipases, lipases and cell wall degrading enzymes (enzymes capable of splitting polysaccharides present in vegetable cell walls). Examples of proteins, homologous to Aspergillus, are phytases, phosphatases, xylanases, β-galactosidases, rennets, glucose oxidases and amylases.

The transcriptional termination regulatory region may be from the gene of interest, the target locus, or any other convenient sequence. Where the construct includes further sequences of interest downstream in the direction of transcription from the gene of interest, the transcriptional termination regulatory region, if homologous with the target locus, should be substantially smaller than the homologous flanking region.

A selection marker is normally employed, which may be part of the expression construct or separate from the expression construct, so that it may integrate at a site different from the gene of interest. Since the recombinant molecules of the invention are preferably transformed to a host strain that can be used for industrial production, selection markers to monitor the transformation are preferably dominant selection markers, i.e., no mutations have to be introduced into the host strain to be able to use these selection markers. Examples of these are markers that enable transformants to grow on defined nutrient sources (e.g. the A. nidulans amdS gene enables A. niger transformants to grow on acetamide as the sole nitrogen source) or markers that confer resistance to antibiotics (e.g., the ble gene confers resistance to phleomycin or the hph gene confers resistance to hygromycin B).

The selection gene will have its own transcriptional and translational initiation and termination regulatory regions to allow for independent expression of the marker. A large number of transcriptional initiation regulatory regions are known as described previously and may be used in conjunction with the marker gene. Where antibiotic resistance is employed, the concentration of the antibiotic for selection will vary depending upon the antibiotic, generally ranging from about 30 to 300 µg/ml of the antibiotic.

The various sequences may be joined in accordance with known techniques, such as restriction, joining complementary restriction sites and ligating, blunt ending by filling in overhangs and blunt ligation, Bal31 resection, primer repair, in vitro mutagenesis, or the like. Polylinkers and adapters may be employed, as appropriate, may be introduced or removed by known techniques to allow for ease of assembly of the expression construct. At each stage of the synthesis of the construct, the fragment may be cloned, analyzed by restriction enzyme digestion, sequencing or hybridization, or the like. A large number of vectors are available for cloning and the particular choice is not critical to this invention. Normally, cloning will occur in E. coli.

The flanking regions may include at least part of the open reading frame of the target locus, particularly the signal sequence, the regulatory regions 5' and 3' of the gene of the target locus, or may extend beyond the regulatory regions. The proteins of the flanking region which comprise the open reading frame will normally not encode an intact gene capable of expression of a functional product. Normally, a flanking region will be at least 100 bp, usually at least 200 bp, and may be 500 bp or more. The flanking regions are selected, so as to disrupt the target gene and prevent its expression. This can be achieved by inserting the expression cassette into the open reading frame proximal to the 5' region, by substituting all or a portion of the target gene with the expression construct, or by having the expression construct intervene between the transcriptional initiation regulatory region at the target locus and the open reading frame. As already indicated, where the termination regulatory region is homologous with the region at the target locus, the 3'-flanking region should be substantially larger than a termination regulatory region present in the construct.

The construct may be transformed into the host as the cloning vector, either linear or circular, or may be removed from the cloning vector as desired. The plasmid will usually be linearized within about 1 kbp of the end of the expression construct. A variety of techniques exist for transformation of filamentous fungi. These techniques include protoplast fusion or transformation, electroporation and microprojectile firing into cells. Protoplast transformation has been found to be successful and may be used with advantage. Mycelium of the fungal strain of interest is first converted to protoplasts by enzymatic digestion of the cell wall in the presence of an osmotic stabilizer such as KCl or sorbitol. DNA uptake by the protoplasts is aided by the addition of CaCl₂ and a concentrated solution of polyethylene glycol; the latter substance causes aggregation of the protoplasts, by which process the transforming DNA is included in the aggregates and taken up by the protoplasts. Protoplasts are subsequently allowed to regenerate on solid medium, containing an osmotic stabilizer and, when appropriate, a selective agent, for which the resistance is encoded by the transforming DNA.

After selecting for transformants, the presence of the gene of interest may be determined in a variety of ways. By employing antibodies, where the expression product is heterologous to the host, one can detect the presence of expression of the gene of interest. Alternatively, one may use Southern or Northern blots to detect the presence of the integrated gene or its transcription product.

The cells may then be grown in a convenient nutrient medium. Low concentrations of a protease inhibitor may be employed, such as phenylmethylsulfonyl fluoride, α2-macroglobulins, pepstatin, or the like. Usually, the concentration will be in the range of about 1 µg/ml to 1 mg/ml. The protease gene(s) may be inactivated in order to avoid or reduce degradation of the desired protein.

Target loci which may be used to advantage are the glucoamylase gene of A. niger or A. awamori, the fungal amylase gene of A. oryzae, the cellobiohydrolase genes of T. reesei, or the acid protease gene of Mucor miehei.

The transformants may be grown in batch or continuous reactors, where the nutrient medium is isolated and the desired product extracted. Various methods for purifying the product, as necessary, may be employed, such as chromatography, e.g., HPLC or preparative thin layer chromatography, solvent-solvent extraction, electrophoresis, combinations thereof, or the like.

The following examples are offered by way of illustration and not by way of limitation.

### EXPERIMENTAL

### Example 1

### Construction of various expression cassettes.

### A. Methodology

All constructs were made using standard molecular biological procedures, as described e.g. in Maniatis et al. (1982) Molecular Cloning, A Laboratory Manual, Cold Spring Harbor Laboratory, N.Y.

### Plasmids pAB6-1, pAB6-2A, pAB6-3 and pAB6-4

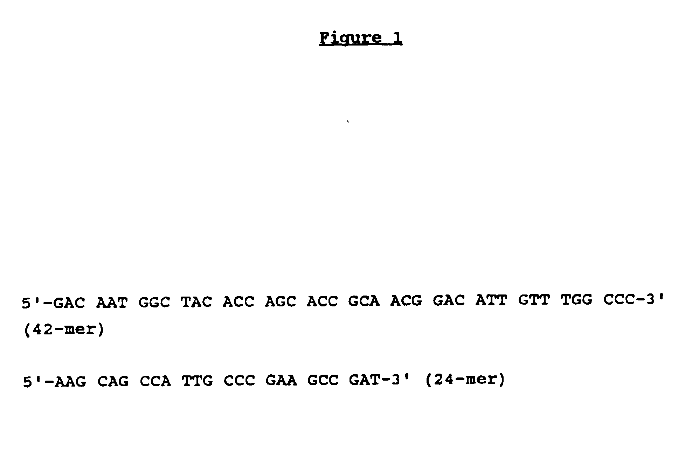

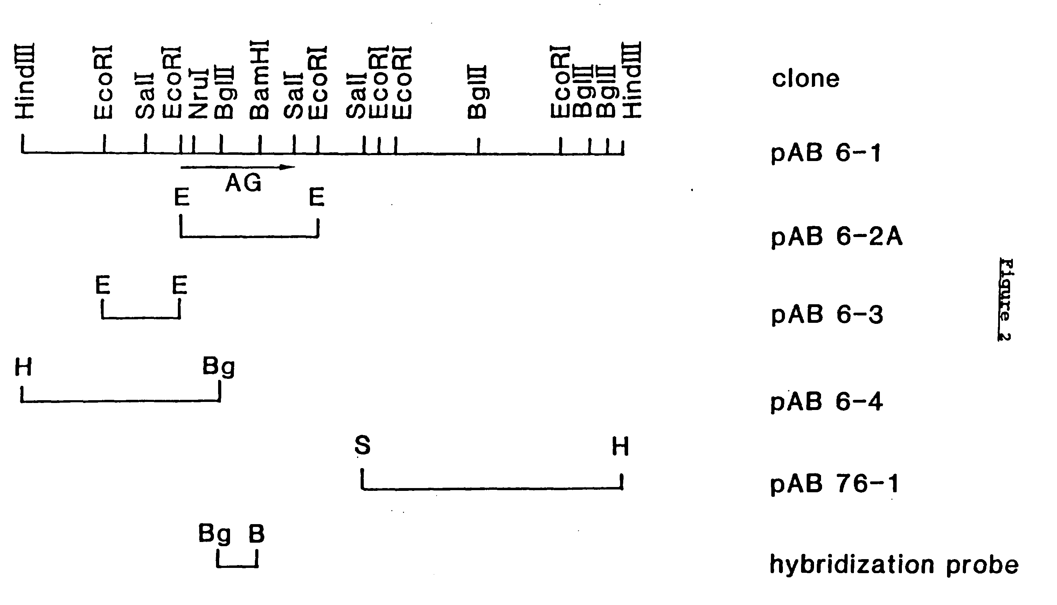

The glucoamylase gene of A. niger was isolated from plasmid libraries, containing 3-4 kb EcoRI fragments or 13-15 kb HindIII fragments in pUC19 (Yanisch-Perron et al., Gene 33 (1985) 103-119, obtainable from e.g. Boehringer Mannheim, Germany), using oligonucleotide probes (Fig. 1) based on the nucleotide sequence published for A. niger (Boel et al., EMBO J. 3 (1984) 1097-1102; Boel et al., Mol. and Cell. Biol. 4 (1984) 2306-2315). The oligonucleotide probes were derived from the sequence surrounding intron 2; the 42-mer is located 3' of the intron and has a polarity identical to the AG-mRNA. The 24-mer is found upstream of intron 2 and is chosen antiparallel to the mRNA. **Plasmid pAB6-1** contains the AG-gene on a 14.5 kb HindIII fragment. In **plasmid pAB6-2A** the entire AG-gene is present on a 3.4 kb EcoRI fragment. **Plasmid pAB6-3** contains the 1.8 kb EcoRI fragment that is present just upstream of the AG gene; this fragment probably contains regulatory sequences and has been subcloned from pAB6-1. **Plasmid pAB6-4**, another subclone of pAB6-1, contains a 4.6 kb HindIII-BglII fragment comprising the region upstream of the AG-gene and part of the 5'-end of the gene (see the map in Figure 2). All fragments were cloned into pUC19.

### Plasmid pAB64-40

Plasmid pAB6-2A (Fig. 2) was treated with AccI and T4 polymerase to generate AccI fragments with blunt ends. The 1.2 kb fragment that contains the 3' flanking sequences of the AG-gene, including the terminator, was isolated. Plasmid pUR1524, containing the bovine chymosin cDNA (see EP-A-077109) was partially digested with SalI and EcoRI. The fragment containing the chymosin cDNA was isolated, sticky ends were filled in with T4-DNA polymerase and the blunt end fragment was ligated with SalI linkers. The fragment was digested with SalI plus HindIII and ligated into pPA153-209 (Van Putten et al., J. Bacteriol. 168 (1986) 728-733) which had been digested with SalI and HindIII. The resulting construct, pRCH4, was cut with BclI and treated with T4 polymerase to generate blunt ends. Subsequently, the AccI fragment containing the AG-terminator, was ligated into this site, yielding **pAB64-10**. In this construct the filled in BclI site, 3' of the chymosin gene is restored.

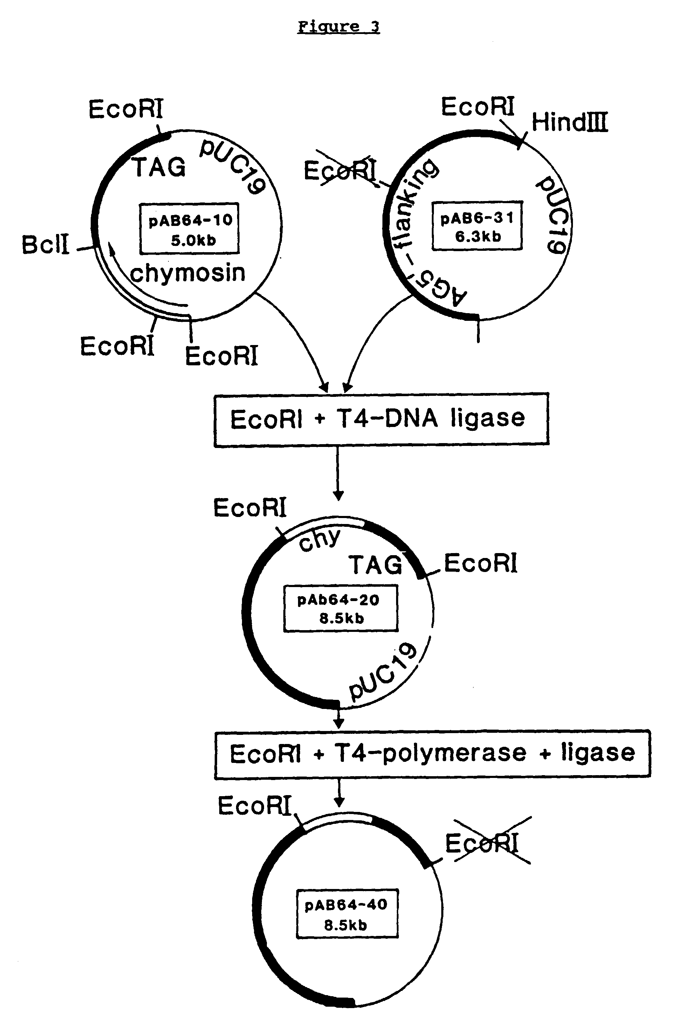

Next, plasmid pAB6-3 (Fig. 2) was partially digested with EcoRI and treated with T4 polymerase. Into this vector the HindIII plus EcoRI fragment of plasmid pAB6-4 was ligated, again after treatment with T4 polymerase. The resulting construct is **pAB6-31**; this construct contains a 3.6 kb upstream fragment of the AG-gene with a destroyed EcoRI site in the middle and a unique EcoRI site close to the AG-gene. In this unique EcoRI site an EcoRI partially digested fragment from pAB64-10, containing the chymosin cDNA plus the AG-terminator was ligated; the resulting construct is **pAB64-20**. The EcoRI site present between the terminator and the vector was destroyed by partial digestion with EcoRI, followed by treatment with T4 polymerase and ligation. The new construct is **pAB64-40**; this construct again contains a unique EcoRI site, now present at the junction of AG-upstream sequences and chymosin cDNA (Fig. 3).

### Plasmid pAN76-1

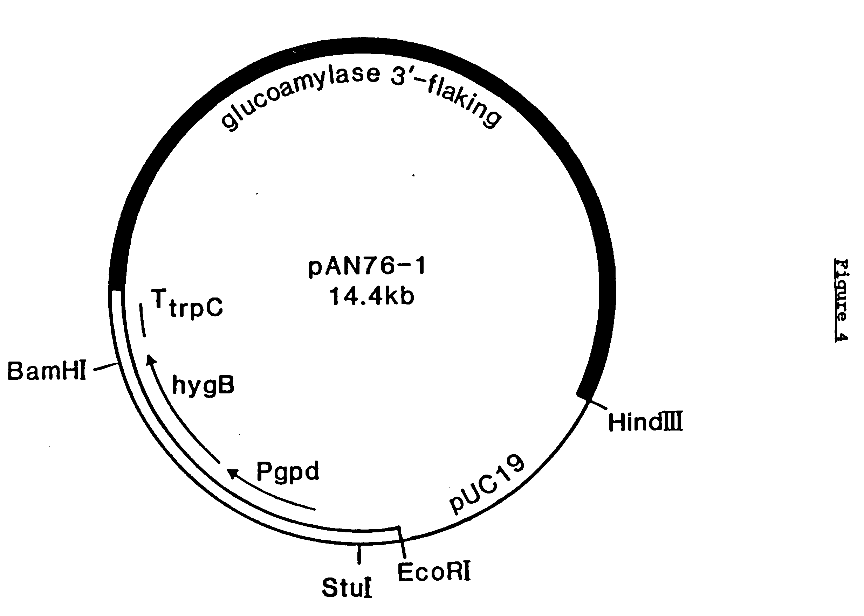

Plasmid pAN7-1 (Punt et al., Gene 56 (1987) 117-124) was digested with HindIII. Into this site the SalI plus HindIII fragment from pAB-6-1 (Figure 2) that is present 3' of the AG-gene was ligated, after filling in the SalI site with T4 polymerase. From this construct, **pAN76-1** (Figure 4), the HygB gene plus the 3' flanking AG fragment was isolated as a StuI plus HindIII fragment.

### Phage mAB64-0

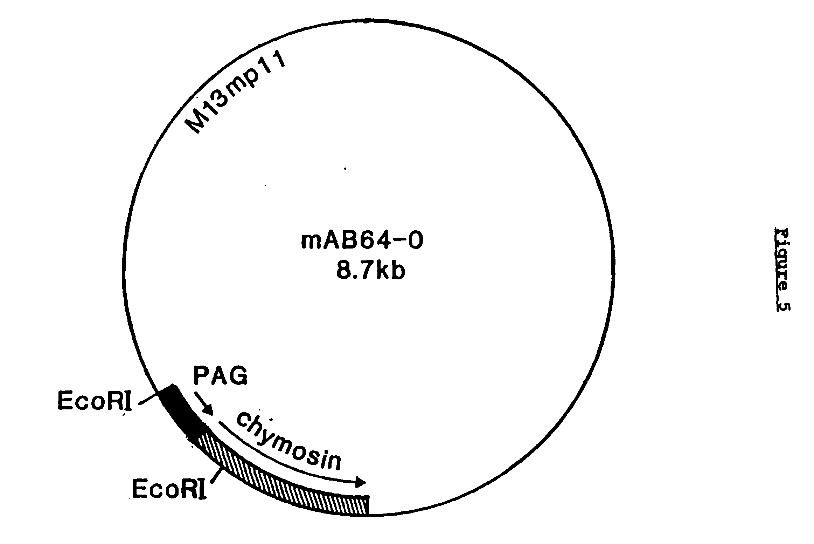

The small EcoRI plus NruI fragment from plasmid pAB6-2A, containing the AG-promoter and regulatory sequences, was isolated and ligated into phage M13mpll (Messing and Vieira, Gene 19 (1982) 269-276, obtainable from e.g. Pharmacia Inc. Sweden) digested with EcoRI and BamHI, together with the SalI plus BclI fragment of plasmid pRCH4, containing bovine chymosin cDNA and treated at the SalI site with T4 polymerase. The resulting construct, **phage mAB64-0**, contains a protein fusion between AG and preprochymosin (Fig. 5); this construct was subjected to in vitro mutagenesis.

### B. Specific expression cassettes

Three expression cassettes for bovine chymosin were constructed from mAB-64-0, each containing a different fusion site between the AG regulatory region and the bovine chymosin cDNA.

To this end the following three oligonucleotides were designed:

#1: 5'- AT TAC ACC TCA GCA ATG AGG TGT CTC GTG GTG 3'
#2: 5'-TGC ACA GGG TTG GCA GCT GAG ATC ACC AGG ATC CCT CT 3'
#3: 5'-ACG GAT AAC CCG GAC GCT GAG ATC ACC AGG 3'

These oligonucleotides were used to join the AG-promoter to preprochymosin (#1) or the AG-promoter and signal peptide to prochymosin (#2). Using oligo #3 a protein fusion is made between amino acid 71 of the mature AG-gene and prochymosin.

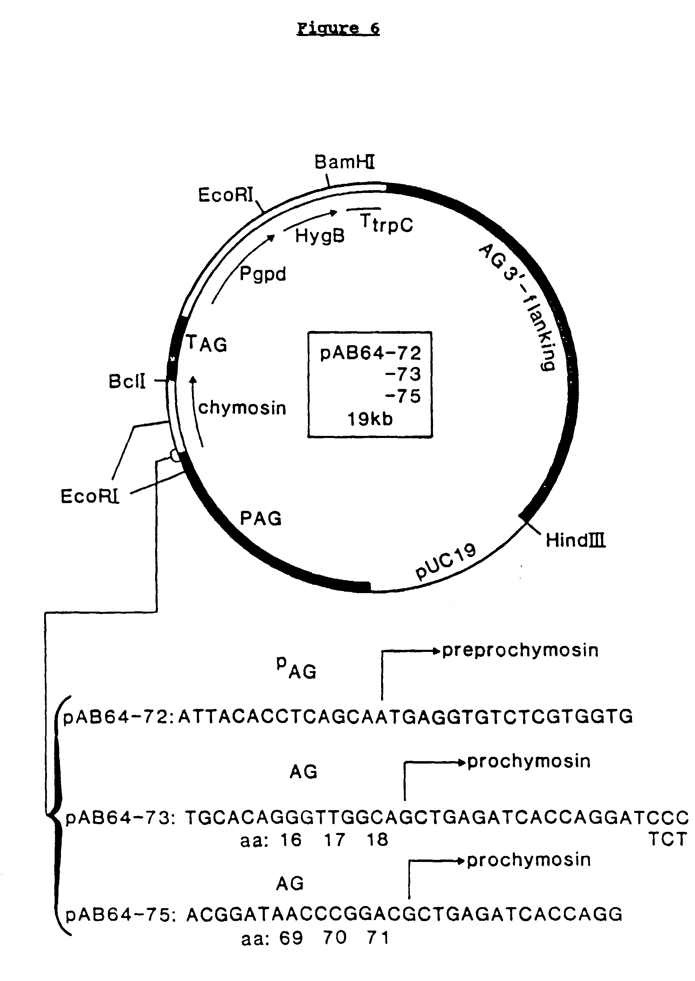

The identity of the constructs was established by sequence analysis of ssDNA of the resulting constructs (mAB64-2, mAB64-3 and mAB64-5). From these constructs the EcoRI fragments containing the desired gene fusions were isolated and inserted into pAB64-40, to obtain pAB64-42, pAB64-43 and pAB64-45, respectively. Into these plasmids pAB64-42, pAB64-43 and pAB64-45 a StuI plus HindIII fragment isolated from pAN 76-1 was inserted, which fragment contained the HygB gene plus a 3'-flanking element of the AG-gene. The final constructs are **pAB64-72**, **pAB64-73** and **pAB64-75**. These constructs contain a unique HindIII site 3' to the expression cassette and 3' flanking region (Fig. 6).

### Example 2

### Transformation of Aspergillus niger to bring about gene replacement

The transformation of A.niger according to the present invention was carried out using techniques as described by Van Hartingsveldt et al. (vide supra) and Yelton et al. (vide supra).

A. niger DS 2975 (a sample of this strain was deposited with CBS under No. 513.88 on August 10, 1988) was grown for 24 hrs. or longer, if required, at 34°C. The mycelium was harvested by filtration through a sterile nylon cloth and washed with 0.6 M MgSO₄, (max. 12.5 ml per 100 ml culture). The mycelium was subsequently transferred to a sterile tube, weighed and resuspended in 5 ml osmotic medium (1.2 M MgSO₄, buffered with 10 mM phosphate, pH 5.8) per gram wet weight. After addition of Novozym 234 (NOVO, Denmark; 20 mg/ml in osmotic medium) at 1 ml per gram of wet weight and BSA (Sigma, Holland; 12 mg/ml in osmotic medium) at 0.5 ml per gram of wet weight, protoplasts were allowed to form while gently shaking (50 rpm) at 25-27°C. Protoplast formation was monitored by microscopic inspection at regular intervals. After protoplast formation was complete, 15 ml of the protoplast solution was transferred to a 30 ml sterile Corex tube and an overlay of 10 ml of trapping buffer (0.6 M sorbitol, 100 mM Tris/HCl pH 7.0) was carefully layered on the protoplast suspension. The tubes were centrifuged at 5000 rpm., 4°C, for 15 min. in a swing-out rotor. Protoplasts were carefully collected from the interphase with a sterile Pasteur pipette with a wide opening, to avoid shearing the protoplasts. The phase separation was repeated when a large pellet was seen after centrifugation; in this case the pellet was resuspended carefully in osmotic medium, a new overlay of trapping buffer applied and centrifugation repeated. Protoplasts were collected and washed carefully by resuspending the protoplasts in cold STC (1.2 M sorbitol; 10 mM Tris/HCl pH 7.5; 50 mM CaCl₂) by resuspending the protoplasts in cold STC and centrifugation at 3000 rpm, 4°C for 10 min. The washing was repeated until a protoplast preparation was obtained that was free from contaminating particles. The protoplast pellet was resuspended in cold STC at a final concentration of 1 x 10⁸ protoplasts (pps)/ml.

Protoplasts were either used immediately or stored overnight at 4°C. If stored, the protoplasts were washed again the next day in cold STC.

For transformation, 10⁷ protoplasts were added to 10 µg of DNA in 25 µl STC or in 10 µl TE (TE contains 10 mM Tris-HCl, 1 mM EDTA, pH 8.0). To obtain gene replacement the plasmid constructs pAB64-72, pAB64-73 and pAB64-75 were linearized by cutting at the unique HindIII restriction site that is present in all three constructs just downstream of the selection marker and 3' flanking region of the AG-gene (Figure 6).

The mixture of protoplasts and DNA was incubated at room temperature for 25 min. Next, PEG-solution (60% PolyEthyleneGlycol 4000, Brocacef/BDH; 10 mM Tris/HCl pH 5.7; 50 mM CaCl₂) was added in portions of 200 µl, 200 µl and 850 µl. The mixture was carefully homogenized after addition of each portion of PEG and incubated at room temperature for 20 min. after addition of the last portion of PEG. Precipitation of large plasmids (>18 kb) sometimes occurred; this was prevented by replacing the 60% PEG-solution by a 25% PEG solution.

The transformed protoplasts were washed with 10-15 ml of cold STC by gentle mixing and centrifugation at 5000 rpm, 4°C for 10 min. The protoplasts were resuspended in 100 µl STC and plated carefully onto selective plates, containing 200 µg/ml of hygromycin B (Sigma H 2638). Hygromycin-resistant colonies were analyzed further, starting from single isolated spores.

### Example 3

### Demonstration of gene replacement

### A. Southern blotting

To establish whether indeed gene replacement had occurred in the transformants, the technique of Southern blotting was used to demonstrate the absence of glucoamylase specific DNA from the transformants. A DNA fragment, homologous to the glucoamylase gene is made radioactive and is hybridized to DNA of the transformants after separation of the DNA by electrophoresis and fixation of the chromosomal DNA to a nylon membrane. Very small amounts of homologous DNA can be detected with this method. DNA was isolated from several transformants of each of the three constructs used for transformation. DNA was isolated after grinding the mycelium in liquid nitrogen, using standard procedures. Genomic DNA was digested with EcoRI, separated by electrophoresis and blotted onto Gene Screen Plus (TM) nylon membrane (Dupont, USA). First, the blots were hybridized with a radioactive AG-specific probe: the AG internal BamHI plus BglII fragment (Figure 2). Transformants that showed no hybridization signal using this probe (a 3.4 kb EcoRI fragment is expected if the AG-gene is still present in the transformants) were screened with other probes to confirm the occurrence of gene replacement. To this end EcoRI digests were hybridized with both radioactive pAB64-75, to confirm the presence of all essential plasmid-derived fragments, and with radioactive pUC19, to confirm the absence of bacterial DNA. The results of these experiments showed the presence of all expected plasmid derived fragments, including the bovine chymosin cDNA, and the absence of bacterial DNA in 7 of the 36 transformants analyzed initially. All selected transformants contain one single chymosin expression cassette.

### B. Western blotting

The absence of a selected gene from the genome of an organism can be established in various ways. Southern blot hybridization, as described in the above paragraph A, is a powerful means to establish the absence of genetic material that is homologous to the probe used. Another way to demonstrate the absence of a specific gene is to demonstrate the absence of the product of the gene of interest, in this case the glucoamylase gene. A very sensitive technique to demonstrate the presence or absence of a protein in a preparation is Western blotting. In this technique specific antibodies are used, raised against the protein of interest, to demonstrate the presence or absence of that specific protein. These antibodies bind to the protein, which had been fixed onto a nitrocellulose membrane. Next, a second antibody preparation is used that is raised against the first antibody preparation. This second antibody preparation is conjugated with an enzyme that is able to process a colourless substrate into a coloured product. A coloured band will appear on those spots where immunogenic material is present that can react with the first antibody preparation.

The transformants that were selected from the Southern blotting results as having the AG-gene replaced by the bovine chymosin gene, were grown in the presence of starch to induce the AG regulatory region and fermentation supernatants were analyzed by Western blotting and compared to a fermentation supernatant of the host strain. 20 µl of each supernatant was layered onto a 7.5% polyacrylamide-SDS gel and protein bands were separated by electrophoresis. The gel was blotted electrophoretically onto a nitrocellulose membrane using standard protocols. The nitrocellulose membrane was incubated, after several washing steps, with a polyclonal antiserum raised against glucoamylase that had been purified from a commercial preparation by HPLC column chromatography. Treatment of the membrane with the second antibody-conjugate preparation, revealed no coloured bands in the transformant preparations, while in the host preparation a band of about the size of glucoamylase was present. This experiment demonstrates that in the selected transformants no material is synthesized that can react with the anti AG-serum, and hence it is concluded that the AG-gene has been replaced by the bovine chymosin gene.

### C. Influence of gene replacement on the amount of secreted protein

Glucoamylase forms a substantial part of the amount of the total protein that is secreted by A. niger (e.g. Cullen et al., vide supra). Therefore, replacement of the glucoamylase gene by another protein gene may influence the amount of protein secreted by A. niger transformants; a decrease in protein content of the fermentation broth may facilitate the purification of other secreted proteins. Moreover, if the capacity of the secretory pathway is limited, the removal of glucoamylase from the cell will leave the secretory pathway open for other proteins.

To investigate the effect of gene replacement on the amount of secreted protein, the protein content of fermentation supernatants was determined by standard methods and compared to the host data. Table 1 summarizes the results obtained.

**Table 1**

| Protein content of fermentation broths (cf. Example 4). | |
|---|---|
| Strain | Protein Concentration (mg/ml) |
| A. niger DS 2975 | 0.143 |
| A. niger DS 2975 + pAB64-72 | 0.096 |
| A. niger DS 2975 + pAB64-73 | 0.082 |
| A. niger DS 2975 + pAB64-75 | 0.076 |

The results obtained in this experiment are consistent with the absence of the highly expressed glucoamylase gene in the transformants analyzed.

### Example 4

### Expression and secretion of bovine chymosin by A. niger using the chymosin and AG-signal peptide

A. niger was transformed with linearized pAB64-72 and pAB64-73 as described in Example 2. The occurrence of gene replacement in the transformants was established as described in Example 3. Selected transformants were analyzed for production and secretion of chymosin, starting from single spore isolates. Chymosin is normally secreted as zymogen: the secreted enzyme is inactive due to the presence of a short N-terminal peptide (prochymosin). This zymogen is activated by removal of the short N-terminal peptide e.g., by enzymatic cleavage or, in the case of chymosin, by incubation at low pH. Therefore, to accurately measure the activity of the secreted chymosin, all the secreted prochymosin is converted to its active form by low pH treatment.

### A. Fermentation of transformants of A. niger containing a gene replacement of the AG-gene

About 10⁷ spores of selected transformants were inoculated into shake flasks, containing 100 ml of liquid preculture medium containing KH₂PO₄ (1 g/l), maltose (30 g/l), yeast extract (5 g/l), hydrolyzed casein (10 g/l), MgSO₄.7H₂O (0.5 g/l) and Tween 80 (3 g/l). These cultures were grown at 34°C for 48 hours. Samples were taken for the analysis of chymosin production and for mRNA isolation; 10 ml of this culture was inoculated into 100 ml of fermentation medium, containing KH₂PO₄ (1 g/l), corn starch (70 g/l), yeast extract (12.5 g/l), hydrolyzed casein (25 g/l), K₂SO₄ (2 g/l), MgSO₄.7H₂O (0.5 g/l), ZnCl₂ (0.03 g/l), CaCl₂ (0.02 g/l), MnSO₄.4H₂O (0.01 g/l), FeSO₄ (0.3 g/l). These cultures were incubated for 48 hrs at 34°C; again samples were taken for the analysis of chymosin production and for the isolation of mRNA.

### B. Determination of mRNA levels in mycelium samples

Mycelium was harvested, washed with distilled water, and ground to a fine powder under liquid nitrogen, using an RNase free mortar and pestle. The powder was dissolved in 2 ml/g of homogenization buffer (4M guanidine isothiocyanate; 5 mM Na citrate pH 7.0; 0.1 M β-mercaptoethanol; 0.5% (w/v) N-lauroyl sarcosine sodium salt (Sigma L-5125)) and incubated at 37°C for 30 min. To the supernatant of a 15 min. 5000 rpm spin 0.4 g/ml of RNase free CsCl was added. This solution was layered carefully onto a 5.7 M CsCl cushion in 0.1 M EDTA; centrifugation was for 17 hr 38000 rpm at 20°C in a Ti 42.1 rotor. The clear RNA pellet was dissolved in RNase free buffer (10 mM Tris-HCl pH 7.4; 5 mM EDTA; 1% SDS) after removal of the guanidine isothiocyanate solution. After extraction with an equal volume of chloroform/butanol (4:1) mixture, the RNA was precipitated with Na acetate and ethanol and dissolved in RNase free water.

RNA samples were electrophoresed and blotted onto Gene Screen Plus (TM) using standard techniques. The blots were hybridized using an oligonucleotide probe, homologous to both the AG and the chymosin mRNA: to this end the oligonucleotide sequence was selected from the AG-leader which is present both in the AG-mRNA and in the chymosin-AG fusion mRNA (in all constructs the AG-chymosin fusion is at the translation start (pAB64-72) or beyond the translation start (pAB64-73 and 75), and hence all transcripts contain the AG-leader).

After cultivation of the mycelium in starch-containing media, hybridizing RNA molecules could be detected in all samples; yields were highest for pAB64-73. In the host, the hybridizing mRNA was of the length expected for the AG-mRNA; the hybridizing mRNA's in the transformants were of the length expected for the various expression cassettes. The mRNA level for AG in the host was equal to the chymosin mRNA level in pAB64-73-transformants, indicating that the hybrid gene at the AG locus in these transformants is transcribed with an efficiency that is equal to that of the original AG-gene.

### C. Demonstration of secretion of bovine chymosin by A. niger

Samples of fermentation broths of both transformants and the host were electrophoresed in a 12.5% polyacrylamide-SDS gel; the gel was subsequently blotted onto nitrocellulose using standard protocols. A monoclonal antibody, raised against purified chymosin, was used for immunological detection of chymosin.

As a control, purified chymosin was also electrophoresed and blotted in the same experiment. The presence of a band of the correct length in all transformant samples, but not in the host sample, indicates expression and secretion of bovine chymosin using either of the two constructs tested. The highest chymosin yield was obtained using constructs pAB64-73, but the secretion of chymosin by pAB64-72 transformants also indicates that the chymosin signal peptide is functional in A. niger.

Clotting activities were determined by standard protocols using dehydrated skim milk powder (DIFCO) as a substrate and calf rennet of known potency as a standard. For pAB64-73, yields ranged between 18 and 45 MCU/ml; pAB64-72 yields were between 7 and 15 MCU/ml. (1 MCU is the amount of chymosin that causes clotting of 1 ml of milk at 35°C in 40 minutes).

The amounts of chymosin obtained in the fermentation broth were up to 11.5 mg/l, which is substantially more than what has been reported for transformants of A. nidulans, which probably bear a greater number of copies of similar chymosin expression cassettes (0.5-2.5 mg/l: Cullen et al. (vide supra) and 0-7 mg/l: EP-A-0215594).

### Example 5

### Expression and secretion of an AG-prochymosin fusion protein from A. niger

Linearized pAB64-75 was transformed to A. niger. Selected transformants were analyzed as described in the previous examples. Chymosin-specific mRNA was demonstrated in the transformants, with an expression level intermediate between pAB64-72 and pAB64-73. Secretion of chymosin by these transformants was demonstrated both by Western blotting and clotting assays; activities ranged between 17 and 29 MCU/ml.

### Example 6

### Regulation of AG-gene expression in the host and of chymosin expression in transformants

The functionality of the AG promoter was assayed in the host and in the selected transformants by growing mycelium in medium containing xylose as the sole carbon source. In this case the AG-promoter is not induced (Nunberg et al., Mol. Cell Biol. 4 (1984) 2306-2315) and expression is expected from neither the AG-gene nor the bovine chymosin gene, which is placed under control of the AG-promoter. Next, mycelium is inoculated into starch containing medium, where induction of the AG-promoter is known to occur (Nunberg et al., vide supra). The predicted induction was demonstrated in Northern blotting experiments by the presence of AG-specific mRNA in the host and of chymosin-specific mRNA in transformants when grown on starch, in contrast to the absence of the specific mRNA's in xylose grown mycelium. Also, secretion of chymosin in induced conditions is demonstrated by either immonoblotting of fermentation broths or by measuring the clotting activity of the fermentation broth. In xylose grown cultures no chymosin activity could be demonstrated by the clotting assay; also results of Western blotting experiments were negative in this case. These experiments demonstrate that regulation of expression of the bovine chymosin gene is similar to that of the AG-gene and is consistent with the current knowledge about expression of the glucoamylase gene.

### Example 7

### Expression of chymosin in the presence of protease inhibitors

The sensitivity of chymosin to protease secreted by A. niger was established by culturing transformants in the presence and absence of protease inhibitors.

To this end transformants of the host strain DS 2975 with construct pAB64-73 (Example 3A) were cultured as described in Example 4. To the culture medium either no protease inhibitors ("-"), or pepstatin and α2-macroglobulin were added ("+") to final concentrations of 1µg/ml and 5µg/ml, respectively. Samples were taken after several time intervals and the amount of chymosin secreted was assayed by Western blot as described in Example 3B. The results are given in Table 2.

**Table 2**

| Relative amounts of chymosin in cultures without ("-") and with ("+") protease inhibitors | | |
|---|---|---|
| Sampling time (hrs after inoculation) | "-" | "+" |
| 7 | -- | - |
| 24 | 1 | 3 |
| 32 | 1 | 3 |
| 48 | 0.5 | 5 |
| 72 | 0.3 | 4 |
| 96 | 0.2 | 2 |

The data indicate that chymosin is sensitive to degradation by protease present in the fermentation broth. Inactivation of these proteases, exemplified by the addition of protease inhibitors, led to an increased yield of chymosin, and judged from the prolonged presence of chymosin in the fermentation broth in the presence of protease, also to an increased stability.

It is evident from the above results, that the subject expression system provides for efficient production of proteins. Furthermore, by introducing the gene of interest into a locus comprising a gene expressing a secretory protein, which protein is produced at high levels, one can provide for the high level production of the gene of interest. In this manner, one reduces the secretory load of the host cell, so as to allow for efficient secretion of the desired product. By initially employing hosts which are efficient in secretion, the subject systems can be used for commercial production of products of interest, such as drugs, commercial enzymes, and the like.

All publications and patent applications cited in this specification are herein incorporated by reference as if each individual publication or patent application were specifically and individually indicated to be incorporated by reference.

## Claims

1. A transformed filamentous fungus host comprising an expression cassette originating by *in vitro* recombination and comprising a transcriptional initiation regulatory region, an open reading frame encoding a signal sequence for secretion in frame with a structural gene of interest and a transcriptional termination regulatory region,
the transformed filamentous fungus host being characterized in that the expression cassette is integrated in a chromosome of the filamentous fungus host at a predetermined target locus comprising a gene whose expression product is secreted to a concentration of at least about 0.1 g/l,
the gene of the target locus being further characterized in that it has been inactivated.

2. A transformed filamentous fungus host according to claim 1, wherein the highly expressed gene in the target locus has been inactivated as a result of the integration of the expression construct.

3. A transformed filamentous fungus host according to claims 1 or 2, wherein the integration of the expression construct at the target locus has occurred through a gene replacement event.

4. A transformed filamentous fungus host according to any one of claims 1 to 3, wherein the host is *Aspergillus.*

5. A transformed filamentous fungus host according to any one of claims 1 to 4, wherein the host is *Aspergillus niger.*

6. A transformed filamentous fungus host according to claim 5, wherein the highly expressed gene in the target locus is the glucoamylase gene.

7. A transformed filamentous fungus host according to any one of claims 1 to 6, wherein the gene of interest encodes a form of chymosin, an interleukin, a phospholipase, a lipase, a phytase, a phosphatase, a xylanase, an amylase, a rennet, a β-galactosidase or a cell wall degrading enzyme.

8. A transformed filamentous fungus host according to any one of claims 1 to 7, wherein the expression construct comprises a marker for selection of hosts comprising said marker.

9. A DNA construct comprising:
a) an expression cassette comprising a transcriptional initiation regulatory region, followed downstream by a gene of interest which is under transcriptional control of the transcriptional initiation regulatory region, followed downstream by a transcriptional termination regulatory region, wherein the gene of interest is provided with a DNA sequence encoding a signal sequence for secretion of the product encoded by the gene of interest,
b) a selectable marker gene for the selection of transformed hosts,
c) 5' and 3' flanking regions homologous to 5' and 3' regions of a predetermined target locus comprising a gene whose expression product is secreted to a concentration of at least about 0.1 g/l, and wherein said locus is endogenous to a filamentous fungus;
said DNA construct being further characterized in that the components under a) and b) are positioned together in between said 5' and 3' flanking regions.

10. A DNA construct according to claim 9, wherein a part of the 5' flanking regions coincides with the transcriptional initiation regulatory region which controls the transcription of the gene of interest.

11. A DNA construct according to claim 10, wherein the coinciding part of the 5' flanking region includes a DNA sequence encoding a signal sequence for secretion of the product of the gene of interest.

12. A DNA construct according to claim 9, wherein a part of the 3' flanking region coincides with the transcriptional termination regulatory region of the gene of interest.

13. A DNA construct according to any one of claims 9 to 12, wherein the endogenous target locus is a glucoamylase gene.

14. A DNA construct according to any one of claims 9 to 13, wherein the gene of interest encodes a form of chymosin, an interleukin, a phospholipase, a lipase, a phytase, a phosphatase, a xylanase, an amylase, a rennet, a β-galactosidase or a cell wall degrading enzyme.

15. A method for producing a protein of interest comprising growing in a nutrient medium a transformed filamentous fungus host as defined in any one of claims 1 to 8.

## Patentansprüche

1. Transformierter filamentöser Pilzwirt, enthaltend eine durch in vitro-Rekombination erhaltene Expressionskassette, die eine transkriptionale Initiationsregulationsregion, einen offenen Leseraster, der für eine Signalsequenz zur Sekretion im Raster mit einem Strukturgen von Interesse kodiert, und eine transkriptionale Terminationsregulationsregion,
wobei der transformierte filamentöse Pilzwirt dadurch gekennzeichnet ist, daß die Expressionskassette in ein Chromosom des filamentösen Pilzwirtes an einem vorbestimmten Ziellocus integriert ist, der ein Gen umfaßt, dessen Expressionsprodukt in einer Konzentration von mindestens etwa 0,1 g/Liter sezerniert wird,
wobei das Gen des Ziellocus ferner dadurch gekennzeichnet ist, daß es inaktiviert worden ist.

2. Transformierter filamentöser Pilzwirt nach Anspruch 1, wobei das in starkem Maße exprimierte Gen im Ziellocus als eine Folge der Integration des Expressionskonstrukts inaktiviert worden ist.

3. Transformierter filamentöser Pilzwirt nach Anspruch 1 oder 2, wobei die Integration des Expressionskonstrukts am Ziellocus durch ein Genaustauschereignis erfolgt ist.

4. Transformierter filamentöser Pilzwirt nach einem der Ansprüche 1 bis 3, wobei es sich beim Wirt um Aspergillus handelt.

5. Transformierter filamentöser Pilzwirt nach einem der Ansprüche 1 bis 4, wobei es sich beim Wirt um Aspergillus niger handelt.

6. Transformierter filamentöser Pilzwirt nach Anspruch 5, wobei es sich beim in starkem Maße exprimierten Gen im Ziellocus um das Glucoamylase-Gen handelt.

7. Transformierter filamentöser Pilzwirt nach einem der Ansprüche 1 bis 6, wobei das Gen von Interesse für eine Form von Chymosin, ein Interleukin, eine Phospholipase, eine Lipase, eine Phytase, eine Phosphatase, eine Xylanase, eine Amylase, ein Rennin, eine β-Galactosidase oder ein zellwandabbauendes Enzym kodiert.

8. Transformierter filamentöser Pilzwirt nach einem der Ansprüche 1 bis 7, wobei das Expressionskonstrukt einen Marker für die Selektion von Wirten, die den Marker enthalten, umfaßt.

9. DNA-Konstrukt, umfassend:
a) eine Expressionskassette, umfassend eine transkriptionale Initiationsregulationsregion, stromabwärts gefolgt von einem Gen von Interesse, das unter der transkriptionalen Kontrolle der transkriptionalen Initiationsregulationsregion steht, stromabwärts gefolgt von einer transkriptionalen Terminationsregulationsregion, wobei das Gen von Interesse mit einer DNA-Sequenz, die für eine Signalsequenz zur Sekretion des durch das Gen von Interesse kodierten Produkts kodiert, versehen ist,
b) ein selektierbares Markergen für die Selektion von transformierten Wirten,
c) 5'- und 3'-flankierende Regionen, die homolog zu 5'- und 3'-Regionen eines vorbestimmten Ziellocus sind, umfassend ein Gen, dessen Expressionsprodukt in einer Konzentration von mindestens etwa 0,1 g/l sezerniert wird, wobei dieser Locus in einem filamentösen Wirt endogen ist,
wobei das DNA-Konstrukt ferner dadurch gekennzeichnet ist, daß die unter a) und b) erwähnten Komponenten zusammen zwischen den 5'- und 3'-flankierenden Regionen positioniert sind.

10. DNA-Konstrukt nach Anspruch 9, wobei ein Teil der 5'-flankierenden Regionen mit der transkriptionalen Initiationsregulationsregion, die die Transkription des Gens von Interesse kontrolliert, zusammenfällt.

11. DNA-Konstrukt nach Anspruch 10, wobei der zusammenfallende Teil der 5'-flankierenden Region eine DNA-Sequenz einschließt, die für eine Signalsequenz zur Sekretion des Produkts des Gens von Interesse kodiert.

12. DNA-Konstrukt nach Anspruch 9, wobei ein Teil der 3'-flankierenden Region mit der transkriptionalen Terminationsregulationsregion des Gens von Interesse zusammenfällt.

13. DNA-Konstrukt nach einem der Ansprüche 9-12, wobei es sich beim endogenen Ziellocus um ein Glucoamylase-Gen handelt.

14. DNA-Konstrukt nach einem der Ansprüche 9-13, wobei das Gen von Interesse für eine Form von Chymosin, ein Interleukin, eine Phospholipase, eine Lipase, eine Phytase, eine Phosphatase, eine Xylanase, eine Amylase, ein Rennin, eine β-Galactosidase oder ein zellwandabbauendes Enzym kodiert.

15. Verfahren zur Herstellung eines Proteins von Interesse, umfassend die Züchtung eines transformierten filamentosen Pilzwirtes gemäß der Definition in einem der Ansprüche 1 bis 8 in einem Nährmedium.

## Revendications

1. Hôte fongique filamenteux transformé comprenant une cassette d'expression provenant d'une recombinaison *in vitro* et comprenant une région régulatoire d'initiation transcriptionnelle, un cadre ouvert de lecture codant une séquence signal pour la sécrétion dans le cadre avec un gène de structure intéressant et une région régulatoire de terminaison transcriptionnelle,
l'hôte fongique filamenteux transformé étant caractérisé en ce que la cassette d'expression est intégrée dans un chromosome de l'hôte fongique filamenteux à un locus cible prédéterminé comprenant un gène dont le produit d'expression est sécrété à une concentration d'au moins environ 0,1 g/l,
le gène du locus cible étant en outre caractérisé en ce qu'il a été inactivé.

2. Hôte fongique filamenteux transformé selon la revendication 1, dans lequel le gène hautement exprimé dans le locus cible a été inactivé comme un résultat de l'intégration de la construction d'expression.

3. Hôte fongique filamenteux transformé selon la revendication 1 ou 2, dans lequel l'intégration de la construction d'expression au locus cible est apparue grâce au remplacement de gènes.

4. Hôte fongique filamenteux transformé selon l'une quelconque des revendications 1 à 3, dans lequel l'hôte est un *Aspergillus.*

5. Hôte fongique filamenteux transformé selon l'une quelconque des revendications 1 à 4, dans lequel l'hôte est un *Aspergillus niger.*

6. Hôte fongique filamenteux transformé selon la revendication 5, dans lequel le gène hautement exprimé dans le locus cible est le gène de la glucoamylase.

7. Hôte fongique filamenteux transformé selon l'une quelconque des revendications 1 à 6, dans lequel le gène intéressant code une forme de chymosine, une interleukine, une phospholipase, une lipase, une phytase, une phosphatase, une xylanase, une amylase, une présure, une β-galactosidase ou une enzyme dégradant la paroi cellulaire.

8. Hôte fongique filamenteux transformé selon l'une quelconque des revendications 1 à 7, dans lequel la construction d'expression comprend un marqueur pour la sélection d'hôtes comprenant ledit marqueur.

9. Construction d'ADN comprenant :
a) une cassette d'expression comprenant une région régulatoire d'initiation transcriptionnelle, suivie en aval par un gène intéressant qui est sous contrôle transcriptionnel de la région régulatoire d'initiation transcriptionnelle, suivie en aval par une région régulatoire de terminaison transcriptionnelle, dans laquelle le gène intéressant est fourni avec une séquence d'ADN codant une séquence signal pour la sécrétion du produit codé par le gène intéressant,
b) un gène marqueur susceptible d'être choisi pour la sélection des hôtes transformés,
c) des régions adjacentes de 5' et 3' homologues des régions 5' et 3' d'un locus cible prédéterminé comprenant un gène dont le produit d'expression est sécrété à une concentration d'au moins environ 0,1 g/l, et dans lesquelles ledit locus est endogène au champignon filamenteux ;
ladite construction d'ADN étant en outre caractérisée en ce que les composants sous a) et b) sont positionnés conjointement entre lesdites régions adjacentes de 5' et 3'.

10. Construction d'ADN selon la revendication 9, dans laquelle une partie des régions adjacentes de 5' coïncide avec la région régulatoire d'initiation transcriptionnelle qui contrôle la transcription du gène intéressant.

11. Construction d'ADN selon la revendication 10, dans laquelle la partie coïncidante de la région adjacente de 5' inclut une séquence d'ADN codant une séquence signal pour la sécrétion du produit du gène intéressant.

12. Construction d'ADN selon la revendication 9, dans laquelle une partie de la région adjacente de 3' coïncide avec la région régulatoire de terminaison transcriptionnelle du gène intéressant.

13. Construction d'ADN selon l'une quelconque des revendications 9 à 12, dans laquelle le locus cible endogène est un gène de la glucoamylase.

14. Construction d'ADN selon l'une quelconque des revendications 9 à 13, dans laquelle le gène intéressant code une forme de chymosine, une interleukine, une phospholipase, une lipase, une phytase, un phosphatase, une xylanase, une amylase, une présure, une β-galactosidase ou une enzyme dégradant la paroi cellulaire.

15. Procédé de production d'une protéine intéressante comprenant la croissance dans un milieu nutritif d'un hôte fongique filamenteux transformé comme défini dans l'une quelconque des revendications 1 à 8.
